# EUROPEAN PATENT SPECIFICATION

(11) **EP 3 004 444 B1**
(45) Date of publication and mention of the grant of the patent: **20.12.2017**
(21) Application number: 14731869.5
(22) Date of filing: 15.05.2014
(51) Int. Cl.: D04H 1/4291, B32B 5/26, D04H 1/485, D04H 1/49, D04H 1/492, D04H 3/00, D04H 3/007, D04H 3/11, D04H 1/4374, D04H 1/498

(54) **NONWOVEN WEB MATERIAL HAVING ENHANCED GLIDE SOFTNESS AND GOOD STRENGTH ATTRIBUTES, AND METHOD FOR MANUFACTURING**
VLIESSTOFF MIT VERBESSERTER WEICHHEIT UND GUTE FESTIGKEIT UND VERFAHREN ZUR HERSTELLUNG
MATÉRIAU NON TISSÉ WEB AYANT UNE BONNE DOUCEUR ET UNE BONNE RÉSISTANCE , ET PROCÉDÉ DE FABRICATION

(30) Priority: 30.05.2013 US 201313905616
(43) Date of publication of application: 13.04.2016
(73) Proprietor: The Procter & Gamble Company, Cincinnati, OH 45202 (US)
(72) Inventor: KANYA, Kevin, Ronald, Cincinnati, Ohio 45202 (US); ISELE, Olaf, Eric Alexander, Cincinnati, Ohio 45202 (US)
(74) Representative: Heide, Ute
(86) International application number: PCT/US2014/038173
(87) International publication number: WO 2014/193660

(56) References cited:
- EP-A1- 2 505 707
- WO-A1-2009/032865
- US-A1- 2003 004 482

## Description

### BACKGROUND OF THE INVENTION

The business of manufacturing and marketing disposable absorbent articles for personal care or hygiene (such as disposable diapers, training pants, adult incontinence undergarments, feminine hygiene products, breast pads, care mats, bibs, wound dressing products, and the like) is relatively capital intensive and highly competitive. To maintain or grow their market share and thereby maintain a successful business, manufacturers of such articles must continually strive to enhance their products in ways that serve to differentiate them from those of their competitors, while at the same time controlling costs so as to enable competitive pricing and the offering to the market of an attractive value-to-price proposition.

One way in which some manufacturers may seek to enhance such products is through enhancements to softness. Parents and caregivers naturally seek to provide as much comfort as they can for their babies, and utilizing products such as disposable diapers that they perceive as relatively soft provides reassurance that they are doing what they can to provide comfort in that context. With respect to other types of disposable absorbent articles that are designed to be applied and/or worn close to the skin, an appearance of softness can reassure the wearer or caregiver that the article will be comfortable.

Thus, manufacturers may devote efforts toward enhancing the softness of the various components used to make such articles, such as various web materials, including nonwoven web materials formed from polymer fibers, and laminates thereof, forming the articles. Such laminates may include, for example, laminates of polymer films and nonwoven web materials forming the backsheet components of the articles.

It is believed that humans' perceptions of softness of a nonwoven web material can be affected by tactile signals, auditory signals and visual signals.

Tactile softness signals may be affected by a variety of the material's features and properties that have effect on its tactile feel, including but not limited to loft, fiber shape, thickness and density, basis weight, microscopic pliability and flexibility of individual fibers, macroscopic pliability and flexibility of the nonwoven web as formed by the fibers, surface friction characteristics, number of loose fibers or free fiber ends, and other features.

Among materials commonly used for manufacturing nonwoven web materials to be used as components of absorbent articles, polypropylene and polyethylene resins are predominant, for reasons of cost, availability, and amenability to processing, *i.e.,* fiber spinning. Between fibers of equal size formed of these two types of polymers, polypropylene fibers tend to have comparatively greater mechanical strength and stiffness, and tend to exhibit comparatively higher coefficients of friction (*i.e.,* tend to have a less slippery feel) with the skin. By comparison, polyethylene fibers tend to have comparatively less mechanical strength and stiffness (*i.e*., more pliability), and tend to exhibit comparatively lower coefficients of friction (*i.e.,* have a more slippery feel) with the skin. Additionally, fibers formed of polyethylene tend to impart a relatively greater degree of visible and tactilely perceptible "fuzz" to a nonwoven web, as a result of comparatively lower fiber and bond strength.

When these fibers are used to form a nonwoven web, they will impart these attributes to the web. Thus, in forming nonwoven web materials, various attempts have been made to impart desirable characteristics of polypropylene and polyethylene components, while compensating for the shortcomings of each. Generally, mechanical strength of the nonwoven web material is desired for dimensional stability in processing in the manufacture of articles, and to impart structural strength to the articles, in which the web material is a component. Pliability and a lower coefficient of friction a may be desired for a pliant and silky feel, and good drape, contributing to perceptions of tactile softness.

An additional factor affecting the relative proportions of each polymer type that may be used is cost. It is believed that, historically, fibers comprising polyethylene resin have tended to be more expensive to produce than those made from polypropylene resin.

While attempts to manufacture nonwoven web materials in various configurations to find a balance of desirable attributes from these and other materials have achieved some success to date, there is always room for improvement.

WO 2009/032865 A1 relates to nonwoven fabric composites comprising layers of spunbond and meltblown nonwoven webs. The composites are prepared by forming or assembling the layers of the composite such that there are two outer layers of spunbond fibers disposed on opposite sides of at least one inner meltblown layer. Each of the two outer layers comprises spunbond, continuous filament fibers comprising polymeric material with certain Melt Flow Rate characteristics. Further, the fibers in each of the outer layers have been partially attenuated after extrusion from a spinneret to an average of no less than about 1.8 denier per filament. The fibers within each of the spunbond layers are also predominately oriented in the machine direction of the nonwoven fabric composite. All layers of the fabric composites are bonded together via thermal, adhesive or ultrasonic bonding means.

US 2003/004482 discloses a laminate made of fibrous layers for use in absorbent articles. The laminate includes an outer layer made of non-woven fabric, which is in contact with the wearer during use of the article, and an inner layer, which two layers are interconnected in a first bonding pattern consisting of separate bonding points.

EP 2505707 A1 is concerned with the use of a hydroentangled nonwoven fabric made of continuous filaments, which does not exhibit openings, as a loop component of a hook and loop fastener system.

### DESCRIPTION OF THE DRAWINGS

Fig. 1 is a schematic cross-section view of a section of nonwoven web material;
Fig. 2a is a schematic side elevation view depicting equipment and components in a process for manufacturing a nonwoven web.
Fig. 2b is a schematic side elevation view depicting equipment and components in a process for hydrojetting a nonwoven web.
Fig. 3 is a perspective view of a disposable diaper shown laid out horizontally in a relaxed state, wearer-facing surfaces up;
Fig. 4 is a plan view of a disposable diaper shown laid out horizontally in a stretched out, flattened state (stretched out against elastic contraction induced by the presence of elastic members), wearer-facing surfaces facing the viewer;
Fig. 5 is a cross section of the diaper depicted in Figs. 3 and 4, taken through line 2-2 in those figures; and
Fig. 6 is a schematic cross section of a portion of a laminate of a polymeric film and a nonwoven web, taken through a path of bond impressions.

### DESCRIPTION OF EXAMPLES

### Definitions

"Absorbent article" refers to devices that absorb and contain body exudates, and, more specifically, refers to devices that are placed against or in proximity to the body of the wearer to absorb and contain the various exudates discharged from the body. Absorbent articles may include diapers, training pants, adult incontinence undergarments and pads, feminine hygiene products, breast pads, care mats, bibs, wound dressing products, and the like. As used herein, the term "exudates" includes, but is not limited to, urine, blood, vaginal discharges, breast milk, sweat and fecal matter.

"Absorbent core" means a structure typically disposed between a topsheet and backsheet of an absorbent article for absorbing and containing liquid received by the absorbent article. The absorbent core may also include a cover layer or envelope. The cover layer or envelope may comprise a nonwoven. In some examples, the absorbent core may include one or more substrates, an absorbent polymer material, and a thermoplastic adhesive material/composition adhering and immobilizing the absorbent polymer material to a substrate, and optionally a cover layer or envelope.

"Bicomponent" refers to fiber having a cross-section comprising two discrete polymer components, two discrete blends of polymer components, or one discrete polymer component and one discrete blend of polymer components. "Bicomponent fiber" is encompassed within the term "Multicomponent fiber." A Bicomponent fiber may have an overall cross section divided into two or more subsections of the differing components of any shape or arrangement, including, for example, coaxial subsections, core-and-sheath subsections, side-by-side subsections, radial subsections, etc.

"Film" - means a skin-like or membrane-like layer of material formed of one or more polymers, which does not have a form consisting predominately of a web-like structure of consolidated polymer fibers and/or other fibers.

"Like chemistry," with respect to two structures formed of polymeric material(s), means that the respective polymeric material(s) of the two structures is/are capable of mixing together at a temperature of 300 °C or lower, to form a mixture that behaves as a single thermodynamic phase.

"Monocomponent" refers to fiber formed of a single polymer component or single blend of polymer components, as distinguished from Bicomponent or Multicomponent fiber.

"Multicomponent" refers to fiber having a cross-section comprising more than one discrete polymer component, more than one discrete blend of polymer components, or at least one discrete polymer component and at least one discrete blend of polymer components. "Multicomponent fiber" includes, but is not limited to, "Bicomponent fiber." A Multicomponent fiber may have an overall cross section divided into subsections of the differing components of any shape or arrangement, including, for example, coaxial subsections, core-and-sheath subsections, side-by-side subsections, radial subsections, etc.

A "nonwoven" or "nonwoven web" is a manufactured sheet or web of directionally or randomly oriented fibers, consolidated and thermally, mechanically or chemically bonded together by friction, fusion, cohesion, hydrogen-bonding, adhesion, or one or more patterns of bonds and bond impressions created through localized compression and/or application of heat or heating energy, or a combination thereof. The term does not include fabrics which are woven, knitted, or stitch-bonded with yarns or filaments. The fibers may be of natural or man-made origin and may be staple or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). The size of the fibers in cross section, which may be substantially circular or non-circular, can also be expressed in denier and ranges from 0.001 denier to 400 denier, or more typically from 0.001 denier to 10 denier. Nonwoven fabrics can be formed by many processes such as meltblowing, spunbonding, solvent spinning, electro spinning, and carding, and bonding using thermal, chemical and mechanical means (e.g., calendering, hydro-entangling, and air-thru-bonding). The basis weight of nonwoven fabrics is usually expressed in grams per square meter (gsm).

### DESCRIPTION

As noted in the Background, generally, polyethylene fibers tend to have a more slippery tactile feel than polypropylene fibers. They also tend to be more pliable, and possess less mechanical (tensile) strength. Thus, it may be desirable to form a web having a surface layer formed of polyethylene fibers to impart a slippery, silky feel to the web. Such a layer, however, may be more susceptible to fraying and pilling (formation of small tangled balls or rolls of fibers) with rubbing wear such as may be encountered, for example, by the outer cover of a disposable diaper during wear. Fraying and pilling may undesirably lead to consumer perceptions of poor quality, and structural weakening of the material.

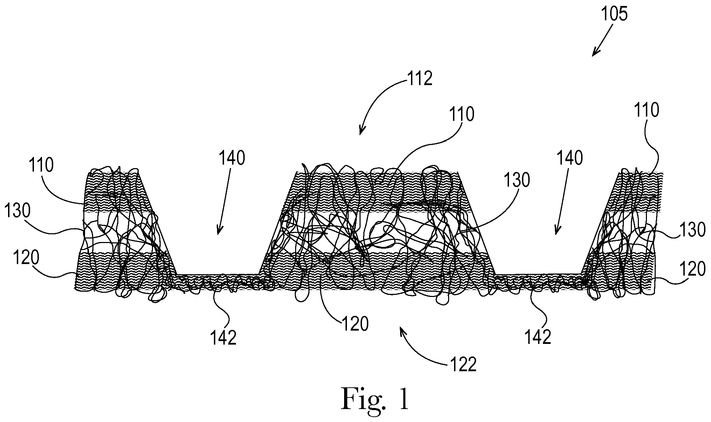

Referring to Fig. 1, one alternative is to form a nonwoven web in a manner that causes fibers having polyethylene components to be interlaced among other components in a manner that exposes fibers having the polyethylene components to the touch at the surface of the web, while at the same time containing/restraining them within the web. Thus, for example, a nonwoven web material 105 may be formed so as to have an first outer accumulation of first fibers 110. The first fibers 110 may be formed of one or more polymers such as polypropylene, selected to impart desirable attributes such as mechanical strength to the web.

The web also may have a second outer accumulation of second fibers 120. The second fibers 120 also may be formed of one or more polymers such as polypropylene, selected to impart desirable attributes such as mechanical strength to the web.

The first and second outer accumulations of respective first and second fibers 110, 120, may be disposed in such a manner as to form respective first and second oppositely-facing macroscopic web outer surfaces 112, 122.

An inner accumulation of third fibers 130 may be included, and may be disposed, to some extent, approximately between the first and second outer accumulations. Through appropriate processing such as described below, third fibers from the inner accumulation of fibers 130 may further be interlaced among the first and second fibers, and may protrude outwardly from the macroscopic web surfaces 112, 122.

The third fibers 130 may be formed entirely of polyethylene, or may include polyethylene components, such that they include surfaces of polyethylene with a desirable slippery feel. In one example, the third fibers 130 may include bicomponent fibers having polyethylene components. The bicomponent fibers may have any suitable configuration by which the polyethylene component is present at, or forms, outer surfaces of the fibers. For example, bicomponent fibers may have a side-by-side arrangement in which polyethylene forms one longitudinal side and longitudinal portion of the outer surface of the fiber. In another example, bicomponent fibers may have a sheath-core arrangement in which polyethylene forms an outer sheath about a core formed of the second component. The second component of the bicomponent fiber may be polypropylene, but may also be another polymer or blend of polymers suitably selected.

The nonwoven web material 105 may bear an impressed pattern of thermal bonds 140. The bonds may be formed by passing a batt of fibers through the nip between a pair of calender/bonding rollers. At least one of the rollers may have formed on its cylindrical rolling surface a pattern of bonding protrusions and recessed areas, where the bonding protrusions are shaped and arranged so as to impress the desired pattern of thermal bonds, with the desired shape(s) in the nonwoven web material. At least one of the rollers may be supplied with heating energy, so that heat will be transferred from the roller(s) to the fibers and cause them to melt and at least partially fuse beneath the bonding protrusions, as the batt passes through the nip.

Thus, in the example depicted in Fig. 1, the respective first fibers 110 and second fibers 120 may be bonded/fused together at the bond sites 142. This effect may be enhanced where first fibers 110 and second fibers 120 are of like chemistry such that they tend to fuse upon melting. Thus, for example, if first fibers 110 and second fibers 120 are identical or suitably similar formulations of polypropylene, respectively, such thermal bonding may be readily achieved. The third fibers 130 may be formed of a polymer having a lower melting point than that of the first and/or second fibers, such that the material forming the third fibers will readily melt and flow aside under pressure imposed by bonding protrusions on a bonding roller, to permit contact and fusion of the first and second fibers at the bond sites, at the selected bonding temperature and/or degree of heating energy supplied to the bonding roller(s).

Other selections of polymers to achieve similar effects can be made. The main polymer component of the first and second fibers can be selected from the general class of polypropylenes, particularly isotactic polypropylenes with a typical fiber-grade melt-flow rate such as ranging from, for example, 10 to 50 g/10min MFR. They may be isotactic PP created with Ziegler-Natta-type catalysts or from metalocene catalysts, commonly abbreviated zn- or m- type i-PP. The third fibers may contain polymer components with a relatively lower softening or melting point than first or second fibers, and can be selected from polyethylene, ethylene-propylene copolymers, or from polypropylenes with lower isotacticity, such as syndiotactic or less stereo-regular polypropylenes; all of which achieves a lower softening and melting point while at the same time providing for a more slippery tactile property on the fiber surface.

The compositions may also include other additives like anti-oxidant packages, pigments and dyes, etc.

An appropriately configured pattern of thermal bonds 140 may serve to cause the first fibers 110 and second fibers 120 to form outer networks of bonded fibers that tend to contain and restrain third fibers 130. At the same time, as explained further below, third fibers 130 may be caused to be interlaced among the first fibers 110 and second fibers 120, and to protrude through the outer macroscopic surfaces formed thereby, so as to be exposed for purposes of imparting a slippery, silky tactile feel to the nonwoven web material.

Fig. 1 also depicts an example of macroscopic first and second surfaces 112 and 122 of a web, on sides formed by the first fibers 110 and second fibers 120, respectively. There are sides to the web and the thermal bond sites 142 that are somewhat layered accumulations of the first fibers 110 on one side and of the second fibers 120 on the other side. The bonds 140 may be impressed into either or both of the first and second macroscopic surfaces, at which the first fibers are thermally bonded to the second fibers.

It is not necessary that the layered accumulations of first fibers 110 and second fibers 120 are similar or identical in loft or thickness, basis weight, fiber size, fiber cross-sectional shape, fiber longitudinal shape, fiber directional bias or orientation, or fiber material composition. For example, the accumulation of second fibers 120 may have a greater density or number of fibers per unit area or unit volume, differently sized fibers, differently shaped fibers, fibers of different composition, etc., compared with the accumulation of first fibers 110. It is preferred, however, that first fibers 110 and second fiber 120 or at least components thereof have like chemistry such that the fibers 110, 120 or portions thereof will readily fuse and form robust bonds at bond sites 142.

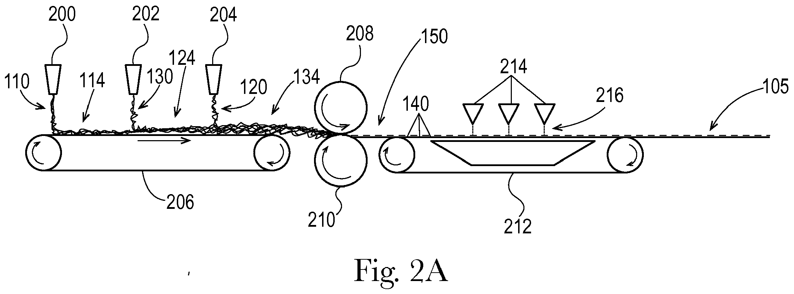

Fig. 2a schematically illustrates an example of equipment configuration and a process by which the nonwoven web material schematically depicted in Fig. 1 may be produced. First fibers 110 may be spun, *e.g.,* in a spunbonding process, at a first beam of spinnerets 200. Following spinning, the first fibers 110 may be directed toward and accumulated on a moving belt 206, forming a first batt 114. Similarly, third fibers 130 may be spun at a third beam of spinnerets 202, and may be directed toward the moving belt such that they accumulate on top of the first batt to form a first combination batt 124. As reflected above, third fibers 130 may be formed of a material differing from that used to form first fibers 110, selected for differing attributes. Also as noted, third fibers 130 may be bicomponent or multi-component fibers. Next, second fibers 120 may be spun at a second beam of spinnerets 204, and may be directed toward the moving belt such that they accumulate on top of the first combination batt 124 to form a second combination batt 134. If it is desired that the second fibers 120 or accumulation thereof be different in some respect than the first fibers 110 or the accumulation thereof, the second beam of spinnerets 204 may be accordingly be configured differently, additional beams of spinnerets may be used, etc. Also as reflected above, first fibers 110 and second fibers 120 may be formed of polymeric materials of like chemistry, for example, similar formulations of polypropylene. Multiple beams 200 or 202 or 204 may be used to form multiple layered accumulations of fibers 110 or 120 or 130 respectively. In one example with a 4 spun bond beam configuration, the first combination batt may include 2 layers of fibers 130 and one layer of fibers 110. Alternatively, 2 beams 204 may be used to effect differing basis weight, loft and/or density of the accumulation of second fibers 120 as compared to the accumulation of first fibers 110.

Following spinning, the second combination batt 134 may be conveyed into the nip between a pair of calender/bonding rollers 208, 210. One or both of the rollers 208, 210 may be supplied with heating energy, so as to be transferred to the fibers in the nip. One or both of the rollers 208, 210 may have formed on the cylindrical rolling surface thereof a pattern of shaped bonding protrusions and recessed areas, such that a like pattern of bonds of like shapes is impressed on the second combination batt 134 as it passes through the nip. Heating energy may be supplied to an extent sufficient to cause melting and fusing of the first and second fibers 110, 120. As a result, a consolidated and bonded web 150, bearing bonds 140, exits the nip.

Next, the bonded web 150 may be conveyed past one or more banks of jets 214 which direct jets 216 of water at the web. (Variants of this process have been called, *e.g*., "hydrojetting," "hydroneedling," "hydroenhancing," "hydroentangling," and "hydroengorging." Examples are described in U.S. Pats. Nos. 6,632,385; 6,803,103; 7,858,544; and RE40,362, and U.S. App. Pub. No. 2012/0179126.) As the web is conveyed past the jets, it may be carried and supported by a suitable belt 212 formed of a permeable mesh. When the water jets 216 impinge the fibers of the bonded web 150, they can displace and tease the fibers and thereby cause the third fibers 130 to be interlaced among the first fibers 110 and second fibers 120. With suitable configurations of jets 214 and water pressures, third fibers 130 may be pushed between and/or among first fibers 110 and/or second fibers 120 and outwardly, to an extent that they are present at the macroscopic web surfaces 112, 122 formed by the first fibers 110 and second fibers 120. In the configuration suggested in Fig. 2a, third fibers 130 will be displaced within the web by the jets 214 so as to be interlaced between and among the fibers of the accumulation of first fibers 110 and be present at the macroscopic surface on the lower side of the web relative the figure, thereby providing a soft-feeling side facing belt 212. In a preferred example, the macroscopic surface of the web 150 bearing the more pronounced depth contrast between bond sites 142 and unbonded areas (*e.g.,* in Fig. 1, macroscopic surface 112) will be the surface facing away from the hydrojets, such that the hydrojets displace the inner softer-feeling fibers toward the surface having the more pronounced surface features, to effect a more visually-impactful and soft-feeling surface on one side, and a relatively smooth, flat and/or less featured surface on the other, more suitable for adhering to another web material to form a web laminate. To achieve this effect, in the example configuration illustrated in Fig. 2a, roller 210 may be an engraved/machined roller bearing the features of the bond pattern, while roller 208 may be a smooth-surfaced (anvil) roller.

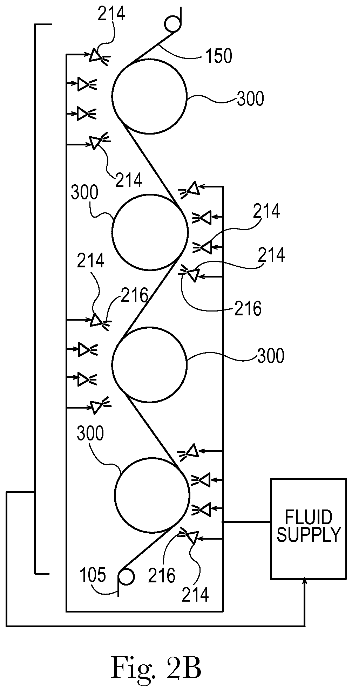

In another alternative, however, the web 150 may be exposed to hydrojetting on both sides, to displaces some of third fibers 130 toward both macroscopic surfaces. As suggested in Fig. 2b, configurations of jets 214, permeable mesh belts and/or permeable surface drums 300, etc. may be arranged such that the bonded web is impinged by jets from both sides, to effect urging of third fibers 130 toward both macroscopic outer surfaces of the web.

Such a hydrojetting step may be employed to displace third fibers 130 within the web such that they are desirably interlaced between and/or among the first fibers 110 and/or second fibers 120, extend through the layers thereof, and are present and exposed to the touch at the first and/or second macroscopic surfaces 112, 122, in a bonded, hydrojetted nonwoven web material as suggested in Fig. 1.

In another alternative, jets of fluid other than water may be directed at the web 150 to displace fibers therewithin and drive fibers inside the fiber matrix among and between other fibers, to cause them to be interlaced therewith and to be present at a macroscopic surface. Jets of gas such but not limited to air may be employed.

In still another alternative, a needlepunching process may be employed to displace fibers within the web structure. Needlepunching involves pushing banks of needles, having forward-oriented barb structures, through the web, in a direction transverse or orthogonal to a macroscopic surface thereof. As the needles advance through the web, the barb structures catch some of the individual fibers within the web and carry them along therewith as the needles advance, driving the caught fibers forward between and among the other fibers in the web, and toward the macroscopic surface facing away from the distal ends of the needles. Needlepunching processes are described in, for example, U.S. Pat. App. Pub. No. 2013/0067706.

In still another alternative, a tufting process as described in, for example, U.S. Pat. 7,553,532, may be employed to displace fibers in an interior layer toward or to an outer macroscopic surface.

It may be noted that thermal bonding and the resulting bonds have the effect of partially locking fibers in place within the web, making it more difficult to displace fibers within the bonded web by hydrojetting or other fiber displacing technique described above, without breaking the bonds and losing some of the benefits of bonding and/or the desirable mechanical strength of the web. In other words, in some circumstances, energy sufficient to displace fibers from an inner layer to the outer macroscopic surfaces may also be sufficient to substantially alter the bonded structure of the web and degrade its mechanical properties (e.g., tensile strength).

However, if the third fibers 130 are formed of a material that has greater ductility than first fibers 110 and/or second fibers 120, when impinged and pushed by hydrojetting or other displacing mechanism they can plastically deform (lengthen) within the web structure to extend out to the macroscopic surfaces, under energy that is not so great as will substantially degrade the web structure. Since polyethylene-based resins are generally more ductile than polypropylene-based resins, forming first and second (*e.g.,* outer) layers of fibers 110, 120 of polypropylene and a third layer of fibers 130 of polyethylene is believed advantageous and thereby suitable for purposes herein. Other combinations of resins having suitable properties and relationships of properties, however, are contemplated, in which outer layers of fiber batts have a first ductility and one or more inner layers of fiber batts have a second, higher ductility. Both displacement and reorientation of fibers (in the thickness direction) as well as a permanent stretching deformation of the fibers or portions of the third fibers are possible mechanisms that lead to the desired effect of having softer-feeling fibers present at a macroscopic surface, while still having them contained and restrained so as not to be subject to undesirable fuzzing and/or pilling.

The enhanced nonwoven web as described herein, and formed as described herein, may be used to form a component of an absorbent article, such as a diaper. For example, the nonwoven web may be used to form a topsheet, leg cuffs and/or backsheet. The nonwoven web may be used as an outer/outward-facing layer of a backsheet, wherein the backsheet is formed of a laminate having layers including a liquid-impermeable film and the nonwoven web.

The enhanced nonwoven web described herein may be hydrophobic or hydrophilic depending on the application and desired functions. When polyolefins are the materials used to form the respective fibers, no further surface treatment is required to make it suitable for use in applications where hydrophobicity is desired, *e.g.,* a hydrophobic backsheet material, or hydrophobic cuff material. When hydrophilicity is desired, however, *e.g*., when the web is to be used as a topsheet material for a diaper or feminine hygiene pad, the web may be treated with a hydrophilic topical application. Alternatively, hydrophilic melt additive can be added to the polymer resin(s) from which the fibers forming the web are spun.

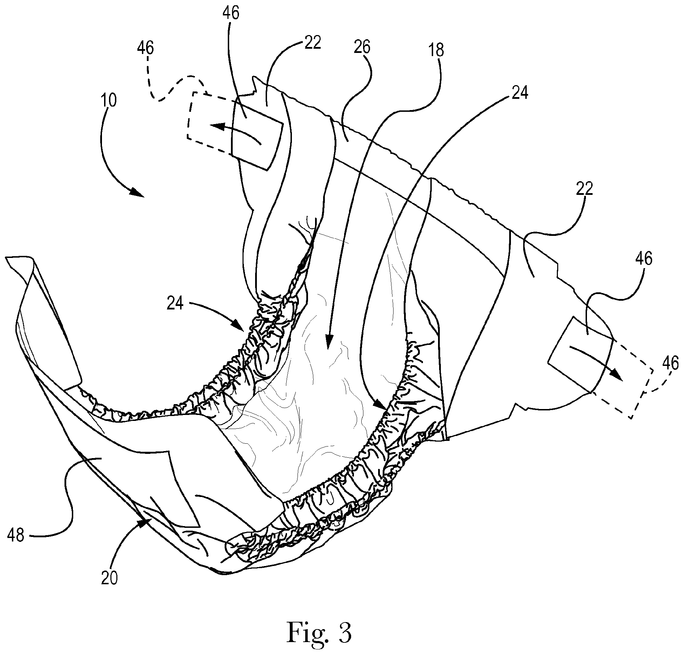

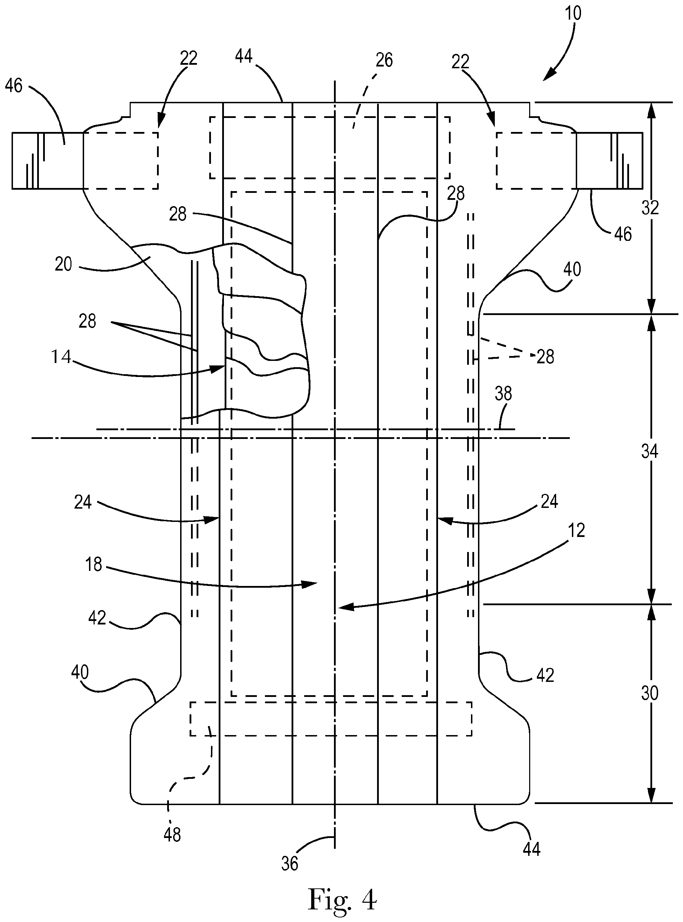

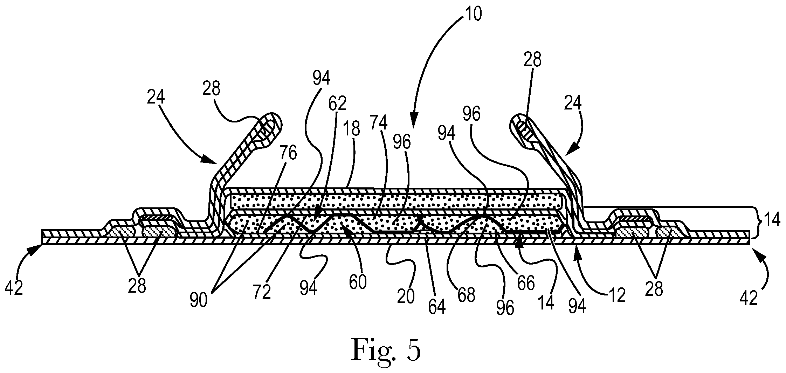

Fig. 3 is a perspective view of a diaper 10 in a relaxed, laid-open position as it might appear opened and lying on a horizontal surface. Fig. 4 is a plan view of a diaper 10 shown in a flat-out, uncontracted state (*i.e.,* without elastic induced contraction), shown with portions of the diaper 10 cut away to show underlying structure. The diaper 10 is depicted in Fig. 4 with its longitudinal axis 36 and its lateral axis 38. Portions of the diaper 10 that contact a wearer are shown oriented upwards in Fig. 3, and are shown facing the viewer in Fig. 4. Fig. 5 is a cross section of the diaper taken at line 2-2 in Fig. 4.

The diaper 10 generally may comprise a chassis 12 and an absorbent core 14 disposed in the chassis. The chassis 12 may comprise the main body of the diaper 10.

The chassis 12 may include a topsheet 18, which may be liquid pervious, and a backsheet 20, which may be liquid impervious. The absorbent core 14 may be encased between the topsheet 18 and the backsheet 20. The chassis 12 may also include side panels 22, elasticized leg cuffs 24, and an elastic waist feature 26. The chassis 12 may also comprise a fastening system, which may include at least one fastening member 46 and at least one landing zone 48.

The leg cuffs 24 and the elastic waist feature 26 may each typically comprise elastic members 28. One end portion of the diaper 10 may be configured as a first waist region 30 of the diaper 10. An opposite end portion of the diaper 10 may be configured as a second waist region 32 of the diaper 10. An intermediate portion of the diaper 10 may be configured as a crotch region 34, which extends longitudinally between the first and second waist regions 30 and 32. The crotch region 34 may include from 33.3% to 50% of the overall length of the diaper 10, and each of waist regions 30, 32 may correspondingly include from 25% to 33.3% of the overall length of the diaper 10.

The waist regions 30 and 32 may include elastic elements such that they gather about the waist of the wearer to provide improved fit and containment (elastic waist feature 26). The crotch region 34 is that portion of the diaper 10 which, when the diaper 10 is worn, is generally positioned between the wearer's legs.

The diaper 10 may also include such other features including front and rear ear panels, waist cap features, elastics and the like to provide better fit, containment and aesthetic characteristics. Such additional features are described in, *e.g.,* U.S. Pats. Nos. 3,860,003 and 5,151,092.

In order to apply and keep diaper 10 in place about a wearer, the second waist region 32 may be attached by the fastening member 46 to the first waist region 30 to form leg opening(s) and an article waist. When fastened, the fastening system carries a tensile load around the article waist.

According to some examples, the diaper 10 may be provided with a re-closable fastening system or may alternatively be provided in the form of a pant-type diaper. When the absorbent article is a diaper, it may comprise a re-closable fastening system joined to the chassis for securing the diaper to a wearer. When the absorbent article is a pant-type diaper, the article may comprise at least two side panels joined to the chassis and to each other to form a pant. The fastening system and any component thereof may include any material suitable for such a use, including but not limited to plastics, films, foams, nonwoven, woven, paper, laminates, stretch laminates, activated stretch laminates, fiber reinforced plastics and the like, or combinations thereof. In some examples, the materials making up the fastening device may be flexible. In some examples, the fastening device may comprise cotton or cotton-like materials for additional softness or consumer perception of softness. The flexibility may allow the fastening system to conform to the shape of the body and thus, reduce the likelihood that the fastening system will irritate or injure the wearer's skin.

For unitary absorbent articles, the chassis 12 and absorbent core 14 may form the main structure of the diaper 10 with other features added to form the composite diaper structure. While the topsheet 18, the backsheet 20, and the absorbent core 14 may be assembled in a variety of well-known configurations, preferred diaper configurations are described generally in U.S. Pat. No. 5,554,145 entitled "Absorbent Article With Multiple Zone Structural Elastic-Like Film Web Extensible Waist Feature" issued to Roe et al. on Sep. 10, 1996; U.S. Pat. No. 5,569,234 entitled "Disposable PuIl-On Pant" issued to Buell et al. on Oct. 29, 1996; and U.S. Pat. No. 6,004,306 entitled "Absorbent Article With Multi-Directional Extensible Side Panels" issued to Robles et al. on Dec. 21, 1999.

The topsheet 18 may be fully or partially elasticized and/or may be foreshortened to create a void space between the topsheet 18 and the absorbent core 14. Exemplary structures including elasticized or foreshortened topsheets are described in more detail in U.S. Pat. No. 5,037,416 entitled "Disposable Absorbent Article Having Elastically Extensible Topsheet" issued to Allen et al. on Aug. 6, 1991; and U.S. Pat. No. 5,269,775 entitled "Trisection Topsheets for Disposable Absorbent Articles and Disposable Absorbent Articles Having Such Trisection Topsheets" issued to Freeland et al. on Dec. 14, 1993. In some examples, the topsheet 18 may be formed of an enhanced nonwoven web 105 as described above.

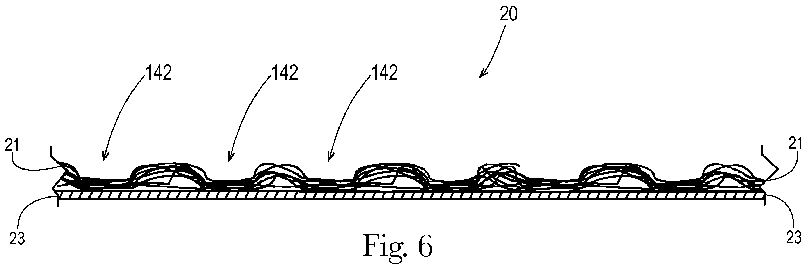

The backsheet 20 may be joined with the topsheet 18. The backsheet 20 may serve prevent the exudates absorbed by the absorbent core 14 and contained within the diaper 10 from soiling other external articles that may contact the diaper 10, such as bed sheets and clothing. Referring to Fig. 6, the backsheet 20 may be substantially impervious to liquids (e.g., urine) and comprise a laminate of a backsheet nonwoven 21 and a thin polymeric film 23 such as a thermoplastic film having a thickness of about 0.012 mm (0.5 mil) to about 0.051 mm (2.0 mils). In some examples, the backsheet nonwoven 21 may be formed of an enhanced nonwoven web 105 as described above. Bond sites 142 at the macroscopic surface having less depth variation between bond sites and unbonded areas (*e.g.,* in Fig. 1, surface 122) may be useful for adhesion to the film via adhesive, in that each site can form a relatively flat, uniform and dense surface area defined by the site, to which adhesive can more readily and uniformly adhere.

Suitable backsheet films include those manufactured by Tredegar Industries Inc. of Terre Haute, Ind. and sold under the trade names X15306, X10962, and X10964. Other suitable backsheet materials may include breathable materials that permit vapors to escape from the diaper 10 while still preventing liquid exudates from passing through the backsheet 20. Exemplary breathable materials may include materials such as woven webs, nonwoven webs, composite materials such as film-coated nonwoven webs, and microporous films such as manufactured by Mitsui Toatsu Co., of Japan under the designation ESPOIR and by EXXON Chemical Co., of Bay City, Texas, under the designation EXXAIRE. Suitable breathable composite materials comprising polymer blends are available from Clopay Corporation, Cincinnati, Ohio under the name HYTREL blend P1 8-3097. Other examples of such breathable composite materials are described in greater detail in PCT Application No. WO 95/16746, published on Jun. 22, 1995 in the name of E. I. DuPont. Other breathable backsheets including nonwoven webs and apertured formed films are described in U.S. Pat. No. 5,571,096 issued to Dobrin et al. on Nov. 5, 1996.

In some examples, the backsheet of the present invention may have a water vapor transmission rate (WVTR) of greater than about 2,000 g/24h/m2, greater than about 3,000 g/24h/m2, greater than about 5,000 g/24h/m2, greater than about 6,000 g/24h/m2, greater than about 7,000 g/24h/m2, greater than about 8,000 g/24h/m2, greater than about 9,000 g/24h/m2, greater than about 10,000 g/24h/m2, greater than about 11,000 g/24h/m2, greater than about 12,000 g/24h/m2, greater than about 15,000 g/24h/m2, measured according to WSP 70.5 (08) at 37.8 0C and 60% Relative Humidity.

## Claims

1. A nonwoven web material, comprising:
a first outer accumulation of fibers (110), formed of first fibers consisting essentially of polypropylene, forming a first macroscopic outer web surface;
a second outer accumulation of fibers (120), formed of second fibers consisting essentially of polypropylene, forming a second macroscopic outer web surface;
an inner accumulation of fibers (130), formed of third fibers comprising polyethylene, the third fibers being interlaced among the first fibers and/or the second fibers and being present at the first and/or second macroscopic web surfaces; and
a pattern of thermal bonds (140) impressed on either or both of the first and second macroscopic surfaces, at which the first fibers are thermally bonded to the second fibers.

2. The nonwoven web material of claim 1, wherein the third fibers are multicomponent fibers comprising a polyethylene component.

3. The nonwoven web material of claim 2, wherein the multicomponent fibers are bicomponent fibers comprising polyethylene and polypropylene components.

4. The nonwoven web material of claim 3, therein the polypropylene components of the bicomponent fibers are at least partially thermally bonded to the first fibers and the second fibers.

5. The nonwoven web material of any of the preceding claims, further comprising a second inner accumulation of fibers, formed of fourth fibers.

6. The nonwoven web material of claim 5 wherein the fourth fibers comprise polyethylene.

7. The nonwoven web material of claim 6 wherein the fourth fibers are multicomponent fibers comprising a polyethylene component.

8. A method for manufacturing a nonwoven web material, comprising the steps of:
spinning first fibers (110) at a first beam of spinnerets, from a first polymeric resin;
directing the first fibers onto a moving belt (206) and accumulating them thereon to form a first batt (114); spinning second fibers (130) at a second beam of spinnerets, from a second polymeric resin differing from the first polymeric resin;
directing the second fibers toward the moving belt and accumulating them over the first batt to form a second batt laid over the first batt, and thereby forming a combination batt (124) comprising the first and second batts;
conveying the combination batt through a nip between a pair of bonding rollers (208, 209), at least one of said rollers being supplied with heating energy, and thereby impressing a pattern of thermal bonds (140) on the combination batt, wherein fibers of the combination batt are thermally bonded together, and thereby forming a bonded web (150); and
conveying the bonded web past a fiber displacing apparatus (214) wherein fibers within the bonded web are displaced relative other fibers within the bonded web,
wherein the fiber displacing apparatus comprises a plurality of water jets (214) wherein the jets impinge on the bonded web and urge the second fibers through spaces between the first fibers and/or through spaces between respective ones of the first fibers.

9. The method of claim 8, further comprising the steps of:
prior to conveying the combination batt through said nip:
spinning third fibers (120) at a third beam of spinnerets, from a third polymeric resin;
and directing the third fibers toward the moving belt and accumulating them over the second batt to form a third batt laid over the second batt, such that the combination batt comprises the first, second and third batts.

10. An absorbent article comprising a liquid-permeable topsheet, a substantially liquid-impermeable backsheet and an absorbent core disposed between the topsheet and backsheet, wherein either the topsheet or the backsheet comprises the nonwoven web of any of claims 1-7.

## Patentansprüche

1. Vliesbahnmaterial, umfassend:
eine erste äußere Ansammlung von Fasern (110), die aus ersten Fasern gebildet sind, die im Wesentlichen aus Polypropylen bestehen und eine erste makroskopische äußere Bahnoberfläche bilden;
eine zweite äußere Ansammlung von Fasern (120), die aus zweiten Fasern gebildet sind, die im Wesentlichen aus Polypropylen bestehen und eine zweite makroskopische äußere Bahnoberfläche bilden;
eine innere Ansammlung von Fasern (130), die aus dritten Fasern gebildet sind, die Polyethylen umfassen, wobei die dritten Fasern zwischen den ersten Fasern und/oder den zweiten Fasern verflochten sind und an den ersten und/oder zweiten makroskopischen Bahnoberflächen vorhanden sind; und
ein Muster von thermischen Bindungen (140), das auf eine oder beide der ersten und zweiten makroskopischen Oberflächen aufgeprägt ist, an denen die ersten Fasern thermisch an die zweiten Fasern gebunden sind.

2. Vliesbahnmaterial nach Anspruch 1, wobei die dritten Fasern Mehrkomponentenfasern sind, die eine Polyethylenkomponente umfassen.

3. Vliesbahnmaterial nach Anspruch 2, wobei die Mehrkomponentenfasern Bikomponentenfasern sind, die Polyethylen- und Polypropylenkomponenten umfassen.

4. Vliesbahnmaterial nach Anspruch 3, wobei die Polypropylenkomponenten der Bikomponentenfasern wenigstens teilweise thermisch an die ersten Fasern und die zweiten Fasern gebunden sind.

5. Vliesbahnmaterial nach einem der vorstehenden Ansprüche, ferner umfassend eine zweite innere Ansammlung von Fasern, die aus vierten Fasern gebildet sind.

6. Vliesbahnmaterial nach Anspruch 5, wobei die vierten Fasern Polyethylen umfassen.

7. Vliesbahnmaterial nach Anspruch 6, wobei die vierten Fasern Mehrkomponentenfasern sind, die eine Polyethylenkomponente umfassen.

8. Verfahren zum Herstellen eines Vliesbahnmaterials, umfassend die folgenden Schritte:
Spinnen erster Fasern (110) aus einem ersten Polymerharz an einem ersten Strahl von Spinndüsen;
Führen der ersten Fasern auf ein sich bewegendes Band (206) und Ansammeln derselben darauf, um eine erste Fasermatte (114) zu bilden; Spinnen zweiter Fasern (130) aus einem zweiten Polymerharz, das sich von dem ersten Polymerharz unterscheidet, an einem zweiten Strahl von Spinndüsen;
Führen der zweiten Fasern auf das sich bewegende Band und Ansammeln derselben über die erste Fasermatte, um eine zweite Fasermatte zu bilden, die über die erste Fasermatte gelegt wird, und dadurch Bilden einer Kombinationsfasermatte (124), welche die ersten und zweiten Fasermatten umfasst;
Fördern der Kombinationsfasermatte durch einen Walzenspalt zwischen einem Paar von Verbundwalzen (208, 209), wobei mindestens eine dieser Walzen mit Heizenergie versorgt wird, und dadurch ein Muster von thermischen Bindungen (140) auf die Kombinationsfasermatte geprägt wird, wobei Fasern der Kombinationsfasermatte thermisch miteinander verbunden werden, und dadurch eine Verbundbahn (150) gebildet wird; und
Fördern der Verbundbahn vorbei an einer Faserverdrängungsvorrichtung (214), wobei Fasern innerhalb der Verbundbahn relativ zu anderen Fasern innerhalb der Verbundbahn verdrängt werden,
wobei die Faserverdrängungsvorrichtung eine Vielzahl von Wasserstrahlen (214) umfasst, wobei die Strahlen auf die Verbundbahn auftreffen und die zweiten Fasern durch Zwischenräume zwischen den ersten Fasern und/oder durch Zwischenräume zwischen jeweiligen der ersten Fasern drängen.

9. Verfahren nach Anspruch 8, ferner umfassend die folgenden Schritte:
vor dem Fördern der Kombinationsfasermatte durch den Walzenspalt:
Spinnen von dritten Fasern (120) an einem dritten Strahl von Spinndüsen aus einem dritten Polymerharz;
und Führen der dritten Fasern zu dem sich bewegenden Band und Ansammeln derselben über die zweite Fasermatte, um eine dritte Fasermatte zu bilden, die über die zweite Fasermatte gelegt wird, sodass die Kombinationsfaser die erste, zweite und dritte Fasermatte umfasst.

10. Absorptionsartikel, umfassend eine flüssigkeitsdurchlässige Oberschicht, eine im Wesentlichen flüssigkeitsundurchlässige Unterschicht und einen Absorptionskern, der zwischen der Oberschicht und der Unterschicht angeordnet ist, wobei entweder die Oberschicht oder die Unterschicht die Vliesbahn nach einem der Ansprüche 1 bis 7 umfasst.

## Revendications

1. Matériau de nappe non tissée, comprenant :
une première accumulation externe de fibres (110), formée de premières fibres constituées sensiblement de polypropylène, formant une première surface externe macroscopique de nappe ;
une deuxième accumulation externe de fibres (120), formée de deuxièmes fibres constituées sensiblement de polypropylène, formant une deuxième surface externe macroscopique de nappe ;
une accumulation interne de fibres (130), formée de troisièmes fibres comprenant du polyéthylène, les troisièmes fibres étant entrelacées parmi les premières fibres et/ou les deuxièmes fibres et étant présentes au niveau des première et/ou deuxième surfaces macroscopiques de nappe ; et
un motif de liaisons thermiques (140) imprimé sur l'une et/ou l'autre des première et deuxième surfaces macroscopiques, au niveau duquel les premières fibres sont thermosoudées aux deuxièmes fibres.

2. Matériau de nappe non tissée selon la revendication 1, dans lequel les troisièmes fibres sont des fibres à multicomposants comprenant un composant de polyéthylène.

3. Matériau de nappe non tissée selon la revendication 2, dans lequel les fibres à multicomposants sont des fibres à bicomposants comprenant des composants de polyéthylène et de polypropylène.

4. Matériau de nappe non tissée selon la revendication 3, dans lequel les composants de polypropylène des fibres à bicomposants sont au moins partiellement thermosoudés aux premières fibres et aux deuxièmes fibres.

5. Matériau de nappe non tissée selon l'une quelconque des revendications précédentes, comprenant en outre une deuxième accumulation interne de fibres, formée de quatrièmes fibres.

6. Matériau de nappe non tissée selon la revendication 5, dans lequel les quatrièmes fibres comprennent du polyéthylène.

7. Matériau de nappe non tissée selon la revendication 6, dans lequel les quatrièmes fibres sont des fibres à multicomposants comprenant un composant de polyéthylène.

8. Procédé de fabrication d'un matériau de nappe non tissée, comprenant les étapes consistant à :
filer des premières fibres (110) au niveau d'un premier faisceau de buses à filer, à partir d'une première résine polymère ;
diriger les premières fibres sur une courroie mobile (206) et les accumuler sur celle-ci pour former un premier nappage (114) ; filer des deuxièmes fibres (130) au niveau d'un deuxième faisceau de buses à filer, à partir d'une deuxième résine polymère qui diffère de la première résine polymère ;
diriger les deuxièmes fibres en direction de la courroie mobile et les accumuler sur le premier nappage pour former un deuxième nappage posé pardessus le premier nappage, et former de ce fait un nappage en combinaison (124) comprenant les premier et deuxième nappages ;
acheminer le nappage en combinaison à travers une ligne de contact entre une paire de rouleaux de liaison (208, 209), au moins un desdits rouleaux étant alimenté en énergie thermique, et imprimer de ce fait un motif de liaisons thermiques (140) sur le nappage en combinaison, dans lequel les fibres du nappage en combinaison sont thermosoudées les unes aux autres, et former de ce fait une nappe liée (150) ; et
acheminer la nappe liée au-delà d'un appareil de déplacement de fibres (214) dans lequel des fibres au sein de la nappe liée sont déplacées par rapport à d'autres fibres au sein de la nappe liée.
dans lequel l'appareil de déplacement de fibres comprend une pluralité de jets d'eau (214) dans lequel les jets arrivent sur la nappe liée et pressent les deuxièmes fibres à travers des espaces entre les premières fibres et/ou à travers des espaces entre les fibres respectives parmi les premières fibres.

9. Procédé selon la revendication 8, comprenant en outre les étapes consistant à :
avant d'acheminer le nappage en combinaison à travers ladite ligne de contact :
filer des troisièmes fibres (120) au niveau d'un troisième faisceau de buses à filer, à partir d'une troisième résine polymère ;
et diriger les troisièmes fibres en direction de la courroie mobile et les accumuler sur le deuxième nappage pour former un troisième nappage posé pardessus le deuxième nappage, de telle sorte que le nappage en combinaison comprend les premier, deuxième et troisième nappages.

10. Article absorbant comprenant une feuille de dessus perméable aux liquides, une feuille de fond essentiellement imperméable aux liquides et une âme absorbante disposée entre la feuille de dessus et la feuille de fond, dans lequel ou la feuille de dessus ou la feuille de fond comprend la nappe non tissée selon l'une quelconque des revendications 1 à 7.
